(19) Europäisches Patentamt / European Patent Office / Office européen des brevets

(11) **EP 1 915 961 A1**

(12) **EUROPEAN PATENT APPLICATION**

(43) Date of publication:
**30.04.2008 Bulletin 2008/18**

(51) Int Cl.:
*A61B 18/14* (2006.01)  *H03K 3/00* (2006.01)

(21) Application number: **06026790.3**

(22) Date of filing: **22.12.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **26.10.2006 US 553310**

(71) Applicant: **Old Dominion University Norfolk VA 23529 (US)**

(72) Inventors:
• **Schoenbach, Karl, H.**
  **Norfolk**
  **VA 23510 (US)**
• **Heeren, Tammo**
  **Norfolk**
  **VA 23502 (US)**

(74) Representative: **Ferreccio, Rinaldo**
  **c/o Botti & Ferrari S.r.l.**
  **Via Locatelli 5**
  **20124 Milano (IT)**

(54) **Apparatus and methods for performing cellular electro-manipulations**

(57)    An intracellular electro-manipulation apparatus for delivery an electric field pulse to a target of one or more biological cells is provided. The apparatus includes a pulse generator that generates an ultrashort electric field pulse, and a pulse delivery system that directs the ultrashort electric field pulse to the target. The apparatus can include a reflected-signal impeder connected between the pulse generator and the pulse delivery system to impede reflection of a signal to the pulse generator when impedance mismatching between the pulse delivery system and pulse generator occurs. Alternatively, or additionally, the apparatus can include a current limiter connected between the pulse generator and the pulse delivery system to limit current between the pulse generator and the pulse delivery system when a high-conductivity condition occurs in the target.

FIG. 1

**Description**

Field of application

The present invention is related to the field of electrical pulse generation, and, more particularly, to devices and procedures for performing cellular electro-manipulation of biological tissue and cells using electrical pulses.

Background of the invention

Intracellular electro-manipulation is performed by applying electric field pulses to targeted biological tissue or cells thereby inducing change in the underlying structure of the tissue or -cells. In general, the application of an electric field to a biological cell causes a buildup of electrical charge at the cell membrane, which is made up of a lipid bilayer and can be considered a dielectric. The result is a voltage change across the cell membrane. A flux of ions through voltage-induced openings or channels in the membrane can occur if the magnitude of the applied electric field is on the order of the resting potential of the cell membrane. This changes the ion concentration close to the cell membrane, and, as a result, causes cell stress.

Depending on the type and form factor of the cell, a sufficiently low voltage can induce stress on the cell that typically lasts only milliseconds, and normally, does not cause permanent damage to the cell. If the strength of the electric field is sufficiently high, however, ion permeability of the cell may last for several hours before returning to a normal state (a reversible breakdown). Indeed, the strength of the electric field may be high enough to permanently breakdown the membrane, in which case cell death occurs.

A form of cell death known as necrosis occurs when a cell swells and the cell membrane ruptures. When the cell membrane ruptures, the release of intracellular contents can damage neighboring cells and cause inflammation in adjacent tissue. Apoptosis, by contrast, is a relatively benign process of cell "suicide." Through this process, a cell shrinks, dissolves its intracellular contents, and activates phagocytosis by neighboring cells.

The ability to initiate cell death via apoptosis in a selective-manner can provide a number of distinct advantages. Selective initiation of apoptosis, for example, could enable the destruction of certain cells while eliminating or mitigating the non-specific damage to surrounding tissue due to inflammation and scarring that typically occurs with necrosis.

Intracellular electro-manipulation provides a mechanism for selectively modifying cells in ways that can lead to apoptosis. The selective modification of cells using intracellular electro-manipulation is described in U.S. Patent No. 6,326,177, which is incorporated herein in its entirety.

The ability to selectively modify cells in ways that induce apoptosis can provide methods for the selective destruction of undesired cells or tissue, such as cancer cells, fat cells, and cartilage cells, while reducing or eliminating damage to- neighboring cells and tissue. As yet, however, there is a need for an intracellular electro-manipulation apparatus that mitigates or eliminates reflections that may occur due to a mismatch between a pulse generator, which provides an electric pulse, and a delivery system capable of directing the pulse to a target.

A basic assumption in performing intracellular electro-manipulation is that the impedance presented to the pulse generator and delivery system is in the kilo-ohm range. If the assumption is correct, the pulse generator and delivery system can be electrically matched such that no pulse reflections occur at the load (i.e., the target). If, however, an impedance mismatch occurs, particularly due to high conductivity of the target, some of the delivered pulse may be reflected back to its source. As a result, a significant amount of energy stored in the pulse generator may then be delivered to the target. Accordingly, there is a need for an intracellular electro-manipulation apparatus capable of mitigating or eliminating reflections that may occur due to impedance mismatching.

There also is a need to- limit current flow if a condition of inordinately high conductivity arises with respect to the target. Under the previously-noted assumption that the impedance presented by the target is in the kilo-ohm range, the current flow induced by the pulse generator and delivery system is less than approximately 20 amperes. Under conditions of high conductivity, however, the current can reach or exceed 400 amperes. Accordingly, there is additionally a need for an intracellular electro-manipulation apparatus capable of limiting current under conditions of high conductivity with respect to the target.

Summary of the invention

The present invention is directed to apparatus and methods for modifying biological cells. A common feature of the different apparatus provided by the invention is that each effects modifications of biological cells by applying ultrashort electrical field pulses to target cells. As applied, such an ultrashort electrical field pulse can have an amplitude and be applied for a duration that is purposely selected to cause a modification of subcellular structures in the targeted cells without causing an irreversible breakdown of the cells' membranes.

One embodiment of the invention is an intracellular electro-manipulation apparatus for providing electric pulse output to biological cells. The apparatus can include a pulse generator that generates an ultrashort electric field pulse. The apparatus also can include a pulse delivery system that delivers the ultrashort electric field pulse to a target comprising one or more biological cells. The apparatus further can include a reflected-signal impeder connected between the pulse generator and the pulse delivery system for impeding reflection of a signal to the pulse generator when impedance mismatching between the pulse delivery system and pulse generator occurs.

Another embodiment is an intracellular electro-manipulation apparatus that, in addition to a pulse generator for generating ultrashort electric field- pulses and a pulse delivery system for delivering the ultrashort electric field pulses to targeted biological cells, can further include a current limiter. The current limiter can be connected between the pulse generator and the pulse delivery system to limit -current between the pulse generator and the pulse delivery system when a high-conductivity condition occurs in the target.

Still another embodiment of the invention is an intracellular electro manipulation apparatus that, in addition to a pulse generator for generating ultrashort electric field pulses and a pulse delivery system for delivering the ultrashort electric field pulses to targeted biological cells, can further include both a reflected-signal impeder and a current limiter. The reflected-signal impeder can be connected between the pulse generator and the pulse delivery system to impede a reflection of a signal to the pulse generator when an impedance mismatching between the pulse delivery system and pulse generator occurs. The current limiter can be connected between the pulse generator and the pulse delivery system so as to limit current between the pulse generator and the pulse delivery system when a high-conductivity condition occurs in the target.

Yet another embodiment of the invention is a method of intracellular electro-manipulation. The method can include generating an ultrashort electric field pulse using a pulse generator. The method also can include delivering the ultrashort electric field pulse to a target using a pulse delivery system, wherein the target comprises one or more biological cells. The method further can include impeding reflection of a signal to the pulse-generator using a reflected-signal impeder when an impedance mismatch between the pulse delivery -system and pulse generator occurs. Additionally, the method can include limiting current between the pulse generator and the pulse delivery system using a -current limiter when a high-conductivity condition occurs in the target.

Brief description of the drawings

There are shown in the drawings, embodiments which are presently preferred, it being understood, however, that the invention is not limited to the precise arrangements and instrumentalities shown.

FIG. 1 is a schematic diagram of an intracellular electro-manipulation apparatus for delivering an electric pulse to a target comprising one or more biological cells, according to one embodiment of the invention.

FIG. 2 is a schematic diagram of an intracellular electro-manipulation apparatus for delivering an electric pulse to a target comprising one or more biological cells, according to another embodiment of the invention.

FIG. 3 is a schematic diagram of an intracellular electro-manipulation apparatus for delivering an electric pulse to a target comprising one or more biological cells, according to yet another embodiment of the invention.

FIG. 4 is a schematic diagram of an intracellular electro-manipulation apparatus for delivering an electric pulse to a target comprising one or more biological cells, according to still another embodiment of the invention.

FIG. 5 is -a flowchart of exemplary steps of a method of intracellular electro-manipulation, according to yet another embodiment of the invention.

Detailed description

As described herein, different embodiments of the invention encompass an apparatus for modifying biological cells by applying ultrashort electrical field pulses to one or more target cells. An apparatus, according to the invention, applies-an ultrashort electrical field pulse for a predetermined duration so as to cause modifications to subcellular structures in the targeted cells without causing an irreversible breakdown of the cells' membranes.

An advantage of selectively modifying the subcellular structures without irreversible breakdown of the cells' membrane is that the selective destruction of certain cells, such as cancerous cells, can be effected without inordinate damage to neighboring cells or surrounding tissue. An advantage of using an ultrashort pulse of the type delivered by the apparatus is that the energy of the pulses can be relatively low. Although- the electrical power of the pulse can be several megawatts, the duration is such that the energy can be so low as to have only a negligible thermal effect on a targeted cell.

The theoretical underpinnings of the invention can be described by considering an equivalent circuit of a biological cell, according to which the cell is modeled as a homogeneous, conductive medium surrounded by a dielectric membrane. Substructures of the cell can be modeled by treating the membrane surrounding the nucleus of the cell as a capacitor and treating the interior of the nucleus as a resistor. Both elements are arranged in series, with the combination of elements, in turn, being in parallel with a resistance representative of the cytoplasm of the cell.

It follows from fundamental principles of electrical circuit analysis that low frequency electric fields will predominately affect the larger capacitance, namely, the outer membrane of the cell. With increasing electric field frequency, the outer membrane will be effectively shorted out, and the applied voltage will appear across the inner (nucleus) membrane. It is predicted, based on this model, that at frequencies around one megahertz the applied voltage should appear mainly across the membrane of the nucleus, rather than across the outer membrane. Accordingly, pulses with shorter pulse widths and- higher frequency components can be expected to affect the nucleus of the cell rather than the cell membrane.

Assuming that the diameter of a targeted intracellular structure, $d$, is small compared to the cell diameter, and -further assuming that the structures are -located at or near the center of the cell, the voltage across the intracellular structure, $V_{is}$, can be modeled according to the equation:

$$V_{is} = E(t)d = j(t)dp_{is} = dp_{is}(E(t)/p_c)\exp\{-t/T_c\},$$

where $p_{is}$ is the resistivity of the target intracellular structure. The charging of the intracellular membrane is predicted to occur with a time constant, $T_{is}$:

$$T_{is} = c_{is}d/2(p_c/2 + p_{is}).$$

The voltage across the intracellular structure membrane, accordingly, is:

$$V_{ism} = V_{is}(1 - \exp(-t/T_{is})) = dp_{is}(E_c/p_c)\exp(-t/T_c)(1 - \exp(-t/T_{is})[u(0) - u(T)].$$

From the theoretical model described, several conclusions can be drawn. A first conclusion is that the voltage across the intracellular membrane can reach values on the same order-as the voltage across the outer membrane, provided that the pulse duration is larger than the charging time of the intracellular membrane and that the pulse rise time is small relative to this charging time.

A second conclusion is that electric field amplitudes in the megavolt/m range are required on a time scale of the charging time of the intracellular membrane in order to reach voltages in excess of one volt across intracellular membranes. For intracellular structures with characteristic dimensions on the order of micrometers, membrane capacitances on the order of microfarads/cm$^2$, and cytoplasm resistivities on the order of 100 $\Omega$-cm, the charging time can be expected to be less- than 10 nanoseconds. The required rate of change of the electric field intensity is -consequently $dE/dt > 10^{14}$ volt/m-sec.

Only if both of these conditions are satisfied can intracellular effects be expected to occur.

A third conclusion is that the voltage across intracellular membranes is expected -to be, at least approximately, linearly dependent on the diameter of the intracellular structure. Stronger effects at larger internal structures would thus be expected with the same electrical parameters.

Reducing the pulse duration, or more specifically, reducing the pulse rise time to values less than the charging time for intracellular membranes, and increasing electric field intensities to the megavolt/m range is predicted to allow preferential targeting of intracellular membranes. Applying a sequence of multiple ultrashort pluses within a relatively short time period can, at least under certain conditions, amplify the effect on intracellular structures without causing substantial defects in the outer surface membrane of a targeted cell.

The different embodiments of the apparatus of the invention described herein employ ultrashort electric field pulses having sufficient amplitude and duration to modify subcellular structures in target cells, at least when applied as a sequence of ultrashort pulses within a relatively short -span of time, such as a sequence of 3-5 ultrashort pulses within a time interval of 10 seconds or less. The amplitude and duration of each ultrashort electric field pulse can be chosen so that it is insufficient to alter permeability of surface membranes of the target cells. The target cells are generally -either in fluid suspension or part of a tissue region. Each ultrashort electric field pulse typically has a pulse duration of no more than about one microsecond and an amplitude of at least approximately 20 kilovolts/cm.

Characterized alternatively, the ultrashort electric field pulses typically have a pulse duration of no more than roughly one microsecond and a total energy of at least about 75 millijoules/cc. More typically, each ultrashort electric field pulse has a total energy of about 75 millijoules/cc to about 2,000 millijoules/cc and, preferably, about 100 millijoules to- about 1,000 millijoules/cc. If extremely short pulse are applied, such as those having durations of about 10 nanoseconds or less, the total energy of the electric field pulse may only be on the order of about 10 to 20 millijoules/cc. In addition to having short durations, the electric field pulses applied have rise times of 50 nanoseconds or less.

The amplitude of the electric field - the applied voltage divided by distance between electrodes - of the pulse is generally at least 20 kilovolts/cm, but preferably does not exceed the breakdown field of the suspension or tissue that includes the targeted cells. The breakdown field increases with decreasing pulse duration. In a typical environment in which the invention can be utilized, the breakdown field generally does not exceed 500 kilovolts. Electric field pulses that are applied for a duration of 10 to 500 nanoseconds typically have amplitudes of about 20 kilovolts/cm to about 300 kilovolts/cm.

To minimize the potential effects on the bulk temperature of the medium (i.e., the thermal effects), the electric field pulses generally have a rapid rise time and short duration. The pulses should preferably be less than one microsecond, but more than 100 picoseconds in duration. A common pulse duration is about one nanosecond to about 500 nanoseconds, with pulses typically having a duration of about 10 to 300 nanoseconds. The optimum pulse duration varies depending to the type of the target cell, tissue type, and desired treatment, as well as other factors. The pulse preferably has a rectangular or trapezoidal shape, but other pulse shapes can be used. For example, in order to open both the outer and inner cell membranes, an intense short pulse might be combined with a less-intense, longer pulse. Other suitable pulse shapes include, for example, exponential decaying pulses, unipolar pulses, and bipolar pulses.

The rise time of the ultrashort electric field pulse is typically no more than about 20 percent. Preferably, the rise time of the ultrashort electric field pulse is no more than about 10 percent of the pulse duration.

The Fourier spectrum of the pulses can include frequencies with substantial amplitudes up to about one gigahertz. Typically, the pulses have Fourier spectra that include frequencies above -one megahertz, with amplitudes greater than 50 percent of the maximum voltage in the spectrum. Preferably, the Fourier spectrum of the pulses includes frequencies between 5 and 50- megahertz, with amplitudes greater than 50 percent of the maximum voltage.

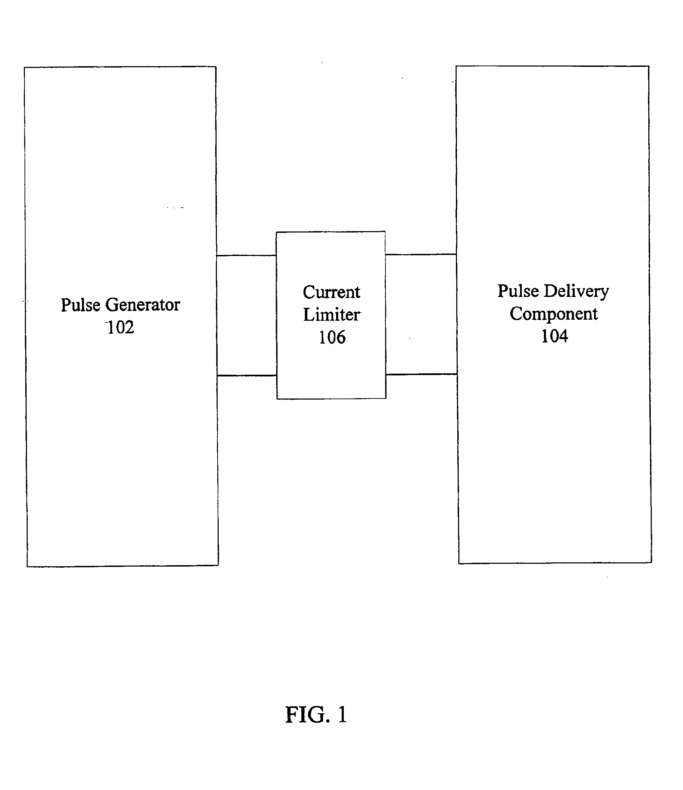

FIG. 1 schematically illustrates an apparatus 100 for delivering ultrashort electric field pulses within a relatively short time interval, according to one embodiment of the invention. The apparatus 100 illustratively includes a pulse generator 102 and a pulse delivery system 104 in electrical communication with one another. The apparatus further illustratively includes a reflected-signal impeder 106 connected between the pulse -generator 102 and the pulse delivery system 104.

More particularly, the pulse generator 102 can comprise a pulse forming network (not explicitly shown) and a high voltage switch (not explicitly shown) connected to the pulse forming network. The pulse forming network can be a high-voltage cable, a strip-line, or a plurality of capacitors and inductors in a transmission line arrangement, as will be readily understood by- one of ordinary skill. The high-voltage switch can be a gaseous, liquid, or solid-state switch.

Energy in the pulse forming network can be stored capacitively, thus requiring a closing switch to release a pulse. Alternatively, energy in the pulse forming network can be stored inductively, requiring an opening switch to release a pulse. In any event, when a switch is triggered, an electrical pulse of the- type already described can be delivered to a load, the load comprising targeted cells in suspension or in tissue. The switch can be triggered by various known mechanisms, including optically or electrically, the latter being effected with a third electrode or by "over-volting" the switch.

The pulse delivery system 104 can comprise a plurality of electrodes between which are positioned the target cells in tissue or a suspension medium. These electrodes may be a solid conducting material, such as a metal, shaped to have one of various geometries, including a planar shape, cylindrical shape, spherical shape, or other geometry. One set of electrodes (not shown) can be connected to the high voltage connection of the pulse generator 102, and a second set of electrodes (also not shown) can be connected to ground, for example, via a stripline or high-voltage cable.

Operatively, the pulse generator 102 generates an ultrashort electric field pulse, the generated pulse having the particular properties described above for performing -intracellular electro-manipulation. The ultrashort electric field pulse generated by the pulse generator 102 is conveyed to the pulse delivery system 104, which delivers the ultrashort electric field pulse to a target comprising one or more biological cells.

As already described, the apparatus 100 can modify subcellular structures in the one or more target cells by delivering to the target a series of ultrashort electric field pulses within a relatively brief time interval. For example, a sequence of three to five ultrashort electric field pulses, whose waveforms have a trapezoidal shape, with durations of 10-300 nanoseconds and amplitudes of 25-300 kilovolts/cm can be delivered to modify intracellular substructures. Multiple pulse sequences with a time interval between pulses of 0.1-3 seconds can be delivered for initiating apoptosis. Although larger numbers of pulses can be applied, the multiple sequences typically include up to about 20 pulses, which are generally spaced at-regular time intervals. Suitable results may be obtained for certain types of cells, such as eosinophils, neutrophils, and T-lymphocytes, by applying three to five ultrashort electric field pulses within a relatively short time period of no longer than five to ten seconds. As also described above, the amplitude and duration of the ultrashort electric field pulses are typically chosen so as to avoid permanently altering the permeability of the surface membrane of the target cells.

It typically can be assumed that the impedance presented to the- pulse generator 102 is in a range of kilo-ohms. If this condition exists, there is- impedance matching between the pulse generator 102 and pulse delivery system 104 range. If, however, the target is in a high-conductivity condition, impedance mismatching may arise. As a result signal- reflection back to the source, the pulse generator 102, could occur.

The reflected-signal impeder 106 is connected between the- pulse generator 102 and the pulse delivery system 104 to mitigate or eliminate problems that might otherwise occur due to impedance mismatching. Specifically, the reflected-signal impeder 106 impedes reflection of a signal to the pulse generator 102 when impedance mismatching of the pulse generator and pulse delivery system occurs. If, for example, a high-conductivity condition develops in the target, giving rise to a risk of impedance mismatching, the reflected-signal impeder 106 can mitigate or eliminate signal reflection back to the pulse generator 102.

Accordingly, with the inclusion of the reflected-signal impeder 106 is connected between the pulse generator 102 and the pulse delivery system 104, a -pulse with positive voltage is delivered to the target during normal pulse delivery. If impedance mismatching occurs, a negative voltage causing reflection back to the source can be prevented by effectively disconnecting the pulse generator 102 and the target. The result is that impedance matching is restored and no further signal reflections are caused.

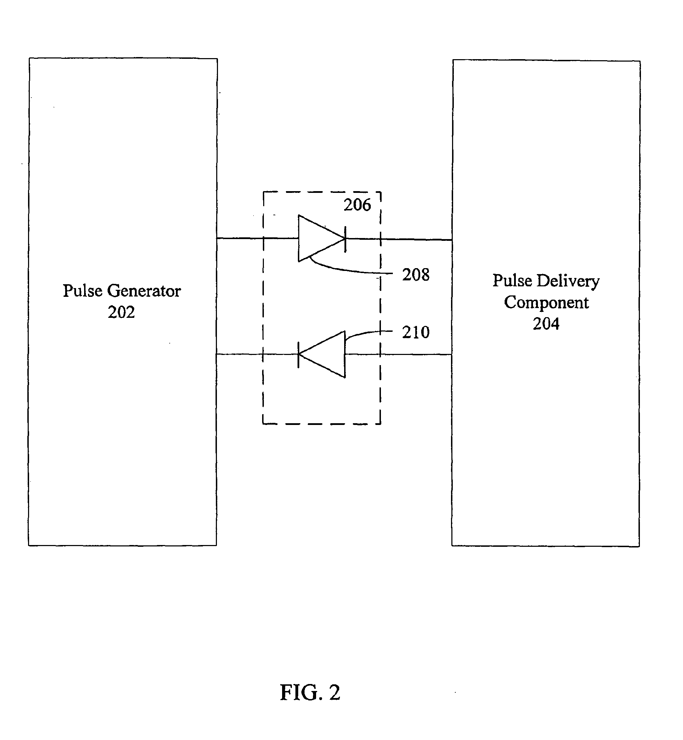

FIG. 2 schematically illustrates a particular embodiment 200 of the invention in which a reflected-signal impeder 206 comprises first and second diodes 208, 210. As illustrated, the reflected-signal impeder 206 is connected between respective signal inputs and outputs of a pulse generator 202 and a pulse delivery system 204.

During a normal pulse delivery operation, a pulse with positive voltage is generated and delivered to the target. The first and second diodes 208, 210 are thus forward biased. The first diode 208 therefore passes electrical current from the pulse generator 202 to the pulse delivery system 204 under the normal operating condition, and the second diode 210 passes electrical current from the pulse delivery system 204 to the pulse generator 202. If impedance mismatching occurs, however, a reflected signal having the opposite, negative polarity is reflected back to the source. The negative polarity, however, ensures that the first and second diodes 208, 210 are each reverse biased.

Accordingly, the first diode 208, when reverse biased, blocks or impedes current from the pulse generator 202 to the pulse delivery system 204. Likewise, the negative polarity reverse biases the second diode 210, which then blocks or impedes current from the pulse delivery system 204 to the pulse generator 202. Thus, again, if impedance mismatching occurs, a negative voltage causing reflection back to the source can be prevented by effectively disconnecting the pulse generator 102 and the target.

One or both of the first and- second diodes 208 and 210 can be a semiconductor device. More particularly, the semiconductor device can have an n-type region formed by doping the semiconductor with an electron-donor material and a p-type region formed by doping the semiconductor with an electron-acceptor material, as will be readily understood by one of ordinary skill. The doping of the respective n-type and p-type regions further can create a pn-junction as will also be readily understood by one of ordinary skill.

Under normal operating conditions, a target comprising tissue or even a single cell is anticipated to present to the intracellular electro-manipulation apparatus an impedance on the order of kilo-ohms. However, if a high-conductivity condition occurs in the target -comprising tissue or even a single cell, the impedance presented by the target may be in the- range of 50 ohms rather than the one or more kilo-ohms anticipated. The apparatus for performing intracellular electro-manipulation under normal operating conditions can induce, for -example, a current through the target of approximately 20 amperes, as described above. If a high-conductivity -condition occurs in the target, however, the current can exceed 400 amperes.

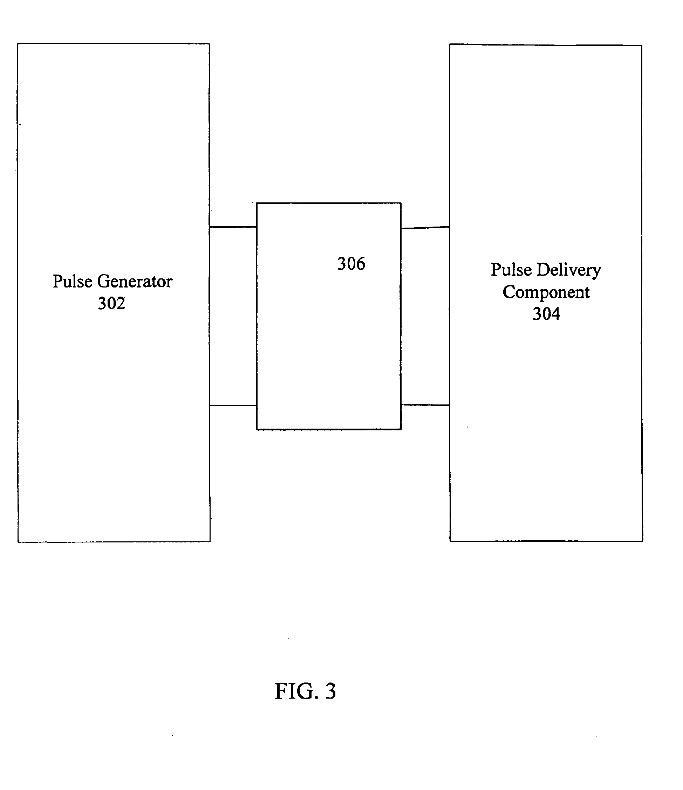

FIG. 3 schematically illustrates another embodiment of an apparatus 300, which according to the invention, limits current in the event that a high-conductivity condition occurs in the target. The apparatus performs intracellular electro-manipulation on a target comprising one or more biological cells by providing electric pulse output to one or more biological cells, as already described. The apparatus 300 illustratively includes a pulse generator 302 and a pulse delivery system 304 in electrical communication with one another. The apparatus 300 also illustratively include a current limiter 306 positioned between and electrically connected with the pulse generator 302 and pulse delivery system 304.

Operatively, the current limiter 306 acts as an artificially induced resistance between the pulse generator 302 and pulse delivery system 304. The current limiter 306 has only a negligible effect on the delivery of an electric field pulse to the target, under normal operating conditions. If a high-conductivity condition occurs within the target, however, the current limiter 306 impedes the current from the pulse generator 302 to the pulse delivery system 304 so as to limit current induced through the target. Preferably, the current limiter 306 reduces the current by 50 percent or more if a high-conductivity condition occurs within the target.

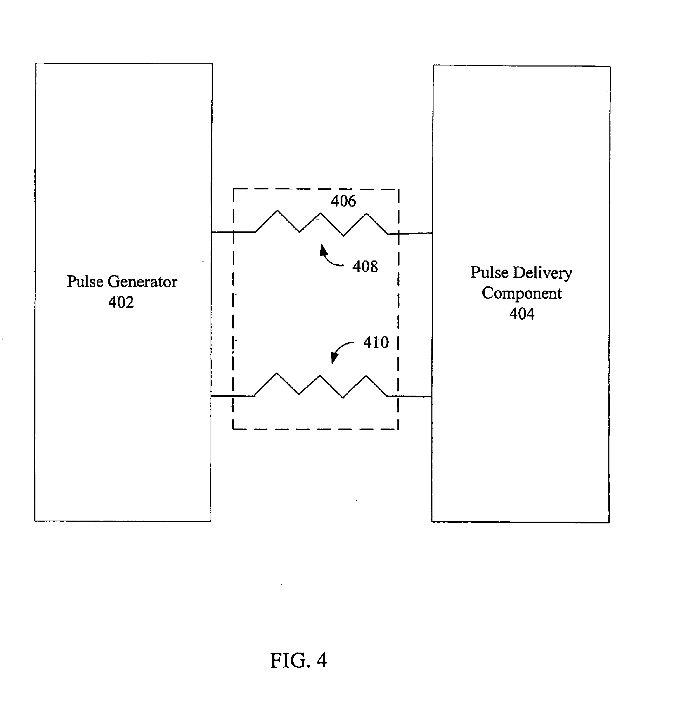

FIG. 4 schematically illustrates a particular embodiment of an apparatus 400 for performing intracellular -electro-manipulation on a target, according to the invention. The apparatus illustratively includes a pulse generator 402 and pulse delivery system 404, with a current limiter 406 connected between the pulse generator and pulse delivery system. According to this embodiment of the invention, the current limiter -406 comprises a first cable, having an artificially induced resistance, 408 connected between the pulse generator 402 and pulse deliver system 404 through which current- is delivered from the pulse generator to the pulse delivery system. As further illustrated, the current limiter 406 also includes a second cable, having an artificially inducted resistance, 410 connected between the pulse generator 402 and pulse deliver system 404 through which current is delivered from the pulse delivery system to the pulse generator. The artificially induced resistance of the first and second cables 408, 410 also suppresses signal reflection that may occur when the target is in a high-conductivity condition, but has no appreciable effect on the deliver of current-to the target under normal operating conditions.

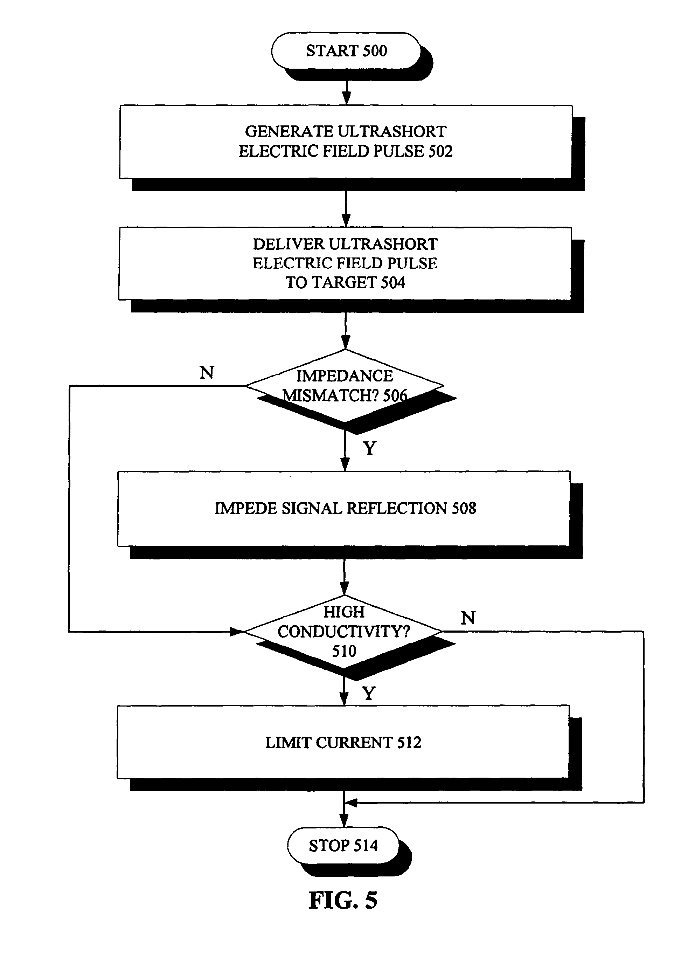

Another embodiment of the invention is a method 500 of intracellular electro-manipulation, as illustrated by the exemplary steps of the flowchart in FIG. 5. The method illustratively includes, at step 502, generating an ultrashort electric field pulse using a pulse generator. The method also illustratively includes delivering the ultrashort electric field pulse to a target using a pulse delivery system, at step 504, the target comprising one or more biological cells.

At decision block 506, it is determined whether an impedance mismatching between the pulse delivery system and pulse generator occurs. If impedance mismatching between the pulse delivery system and pulse generator occurs, then at step 508 reflection of the signal to the pulse generator is impeded using a reflected-signal impeder. Otherwise the method 500 proceeds to decision block 510, where it is determined whether a high-conductivity condition occurs-in the target. If a high-conductivity condition occurs- in the target, then at step 512 current between the pulse generator and the pulse delivery system is limited using a current limiter. Otherwise the method 500 proceeds to step 514. The method illustratively concludes at step 514.

The foregoing description of preferred embodiments of the invention have been presented for the purposes of illustration. The description is not intended to limit the invention to the precise forms disclosed. Indeed, modifications and variations will be readily apparent from the foregoing description. Accordingly, it is intended that the scope of the invention not be limited by-the detailed description provided herein.

**Claims**

1. An intracellular electro-manipulation apparatus for providing electric pulse output to biological cells, the apparatus comprising:

   a pulse generator that generates an ultrashort electric field pulse;
   a pulse delivery system that delivers the ultrashort electric field pulse to a target comprising one or more biological cells; and
   a reflected-signal impeder connected between the pulse generator and the pulse delivery system for impeding reflection of a signal to the pulse generator when impedance mismatching between the pulse delivery system and pulse generator occurs.

2. The apparatus as defined in Claim 1, wherein the reflected-signal impeder comprises a diode having an anode connected -to the pulse generator and a cathode connected to the pulse delivery system to pass electrical current from the pulse generator to the pulse delivery system under a predetermined normal condition and to impede current from the pulse delivery system to the pulse generator when impedance mismatching between the pulse delivery system and pulse generator occurs.

3. The apparatus as defined in Claim 1, wherein the reflected-signal impeder comprises a diode having an anode connected to the pulse delivery system and a cathode connected to the pulse generator to pass electrical current from the pulse delivery system pulse generator to the pulse generator under a predetermined normal condition and to impede current from the pulse delivery system to the pulse generator when impedance mismatching between the pulse delivery system and pulse generator occurs.

4. The apparatus as defined in Claim 1, wherein the at least one diode comprises a first diode and a second diode connected between the pulse generator and the pulse delivery system, the first diode for passing electrical current from the pulse generator to the pulse delivery system under a predetermined normal condition and impeding current from the pulse-delivery system to the- pulse generator when impedance mismatching between the pulse -delivery system and pulse generator occurs, and the second diode passing electrical current from the pulse delivery system to the pulse generator under a predetermined normal condition and impeding current from the pulse delivery system to -the pulse generator when impedance mismatching between the pulse delivery system and pulse generator occurs.

5. The apparatus as defined in Claim- 4, wherein at least one- of the first and second diodes comprises a semiconductor device having an N-type region, a P-type region, and a PN junction positioned between the -N-type and P-type regions.

6. The apparatus as defined in Claim 1, wherein the reflected-signal impeder connected between the pulse generator and the pulse delivery system impedes reflection of a signal to the pulse generator when a high-conductivity condition in the target occurs.

7. An intracellular electro-manipulation apparatus for providing electric pulse output to biological cells, the apparatus comprising:

a pulse generator that generates an ultrashort electric field pulse;
a pulse delivery system -that delivers the ultrashort electric field pulse to a target comprising one or more biological cells; and
a current limiter connected between the pulse generator and the pulse delivery system to limit current between the pulse generator and the pulse -delivery system when a high-conductivity condition occurs in the target.

8. The apparatus defined in Claim 7, wherein the current limiter comprises a connector that induces resistance when- the target is in a high-conductivity -condition so as to limit current from the pulse generator to the pulse delivery system.

9. The apparatus as- defined in Claim 7, wherein the- current limiter comprises- a connector that induces resistance -when the target is in a high-conductivity condition so as to limit current from the pulse delivery system to the pulse generator.

10. The apparatus as defined in Claim 7, wherein the current limiter comprises a first connector that induces a first resistance when the target is in a high-conductivity condition so as to limit current from the pulse generator to the pulse delivery system, -and a second connector that induces a second resistance when the target is in a high-conductivity condition so as- to limit current from the pulse delivery system to the pulse generator.

11. An intracellular electro-manipulation apparatus for providing electric pulse output to biological cells, the apparatus comprising:

a pulse generator that generates an ultrashort electric field pulse;
a pulse delivery system that delivers- the ultrashort electric field pulse to a target comprising one or more biological cells;
a reflected-signal impeder connected between the pulse generator and the pulse delivery system for impeding reflection of a signal to the pulse generator when impedance mismatching between the pulse delivery system and pulse generator occurs; and
a current limiter connected between the pulse generator and the pulse delivery system to limit current between the pulse generator and the pulse delivery system when a high-conductivity condition occurs in the target.

12. The apparatus as defined in Claim 11, wherein the reflected-signal impeder comprises a diode having an -anode connected to the pulse generator and a cathode -connected to the pulse delivery system to pass electrical current from the pulse generator to the pulse delivery system under a predetermined normal condition and to impede current from the pulse delivery system to the pulse generator when impedance mismatching between the pulse delivery system and pulse generator occurs.

13. The apparatus as defined in Claim 11, wherein the reflected-signal impeder comprises a diode having an anode connected to the pulse delivery system and a cathode connected to the pulse generator to pass electrical current from the pulse delivery system pulse generator to the pulse generator under a predetermined normal condition and to impede current from the pulse delivery system to the pulse generator when impedance mismatching between the pulse delivery system and pulse generator occurs.

**14.** The apparatus as defined in Claim 11, wherein the at least one diode comprises a first diode and a second diode connected between the pulse generator and the pulse -delivery system, the first diode for passing electrical current from the pulse generator to the pulse delivery system under a predetermined normal condition and impeding current from the pulse delivery system to the pulse generator when impedance mismatching between the pulse delivery system and pulse generator occurs, and the second diode passing electrical current from the pulse delivery system to the pulse generator under a predetermined normal condition and impeding current from the pulse delivery system to the pulse generator when impedance mismatching between the pulse delivery system and pulse generator occurs.

**15.** The apparatus as defined in Claim 14, wherein at least one of the first and second diodes comprises a semiconductor device having an N-type region, a P-type-region-, and a PN junction positioned between the N-type and P-type regions-.

**16.** The apparatus as defined in Claim 11, wherein the reflected-signal impeder connected between the pulse generator -and the pulse delivery system impedes reflection of a signal to the pulse generator when a -high-conductivity condition in the target occurs.

**17.** The apparatus defined in Claim 11, wherein the current limiter comprises a connector that induces resistance when the target is in a high-conductivity condition so as to limit current from the pulse generator to the pulse delivery system.

**18.** The apparatus as defined in Claim 11, wherein the current limiter comprises a connector that induces resistance when the target is in a high-conductivity condition so as to limit current from the pulse delivery system to the pulse generator.

**19.** The apparatus as defined-in Claim 11, wherein the current limiter comprises a first connector that induces a first resistance when the target is in a high-conductivity condition so as to limit current from the pulse generator to the pulse delivery system, and a second connector that induces a second resistance when the target is in a high-conductivity condition so as to limit current from the pulse delivery system to the pulse generator.

**20.** A method of intracellular electro-manipulation, the method comprising:

generating an ultrashort electric field pulse using a pulse generator;
delivering the ultrashort electric field pulse to a target using a pulse delivery system, the target comprising one or more biological cells;
if impedance mismatching between the pulse delivery system and pulse generator occurs, impeding reflection of a signal to the pulse generator using a reflected-signal impeder; and
if a high-conductivity condition occurs in the target, limiting current between the pulse generator and the pulse delivery system using a current limiter.

```
┌──────────────────────┐        ┌─────────────┐        ┌──────────────────────┐
│                      │        │             │        │                      │
│                      │        │             │        │                      │
│                      │        │             │        │                      │
│                      │        │   Current   │        │                      │
│   Pulse Generator    │        │   Limiter   │        │   Pulse Delivery     │
│        102           │        │    106      │        │    Component         │
│                      │        │             │        │      104             │
│                      │        │             │        │                      │
│                      │        │             │        │                      │
│                      │        └─────────────┘        │                      │
│                      │                               │                      │
└──────────────────────┘                               └──────────────────────┘
```

## FIG. 1

Pulse Generator
202

206

208

210

Pulse Delivery
Component
204

FIG. 2

Pulse Generator
302

306

Pulse Delivery
Component
304

FIG. 3

| Pulse Generator 402 | | Pulse Delivery Component 404 |

406

408

410

FIG. 4

```
                    ┌─────────────────┐
                    │    START 500    │
                    └─────────────────┘
                             │
                             ▼
        ┌──────────────────────────────────────┐
        │        GENERATE ULTRASHORT            │
        │     ELECTRIC FIELD PULSE 502          │
        └──────────────────────────────────────┘
                             │
                             ▼
        ┌──────────────────────────────────────┐
        │        DELIVER ULTRASHORT             │
        │        ELECTRIC FIELD PULSE           │
        │          TO TARGET 504                │
        └──────────────────────────────────────┘
                             │
                             ▼
                  N       ╱──────────╲
        ┌─────────────── ╱  IMPEDANCE  ╲
        │                ╲ MISMATCH? 506╱
        │                 ╲──────────╱
        │                      │ Y
        │                      ▼
        │       ┌──────────────────────────────────────┐
        │       │     IMPEDE SIGNAL REFLECTION 508      │
        │       └──────────────────────────────────────┘
        │                      │
        │                      ▼
        │                   ╱──────────╲
        │                  ╱    HIGH     ╲      N
        └───────────────→ ╲ CONDUCTIVITY? ╱─────────────┐
                           ╲    510      ╱              │
                            ╲──────────╱                │
                                 │ Y                    │
                                 ▼                      │
           ┌──────────────────────────────────────┐    │
           │          LIMIT CURRENT 512            │    │
           └──────────────────────────────────────┘    │
                             │←──────────────────────────┘
                             ▼
                    ┌─────────────────┐
                    │    STOP 514     │
                    └─────────────────┘
```

**FIG. 5**

**EP 1 915 961 A1**

**European Patent Office**

**EUROPEAN SEARCH REPORT**

**Application Number**

EP 06 02 6790

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2004/106917 A1 (ORMSBY THEODORE C [US] ET AL) 3 June 2004 (2004-06-03) * figures 3,4 * | 1,7,11, 20 | INV. A61B18/14 H03K3/00 |
| A | WO 03/060462 A (DUNE MEDICAL DEVICES LTD [IL]; HASHIMSHONY DAN [IL]) 24 July 2003 (2003-07-24) * claim 21; figures 8,10 * | 1,7,11, 20 | |
| A | US 2005/143726 A1 (BORTKIEWICZ ANDRZEJ [US] BORTKIEWICZ ANDZREJ [US]) 30 June 2005 (2005-06-30) * paragraph [0042] - paragraph [0044]; figure 3 * | 1,7,11, 20 | |

**TECHNICAL FIELDS SEARCHED (IPC)**

A61B
H03K

The present search report has been drawn up for all claims

| Place of search | Date of completion of the search | Examiner |
|---|---|---|
| Munich | 11 July 2007 | Brown, Julian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 02 6790

This annex lists the patent family members relating to the patent documents cited in the above-mentioned European search report.
The members are as contained in the European Patent Office EDP file on
The European Patent Office is in no way liable for these particulars which are merely given for the purpose of information.

11-07-2007

| Patent document cited in search report | | Publication date | Patent family member(s) | | Publication date |
|---|---|---|---|---|---|
| US 2004106917 | A1 | 03-06-2004 | NONE | | |
| WO 03060462 | A | 24-07-2003 | AU | 2002360209 A1 | 30-07-2003 |
| | | | DE | 02795418 T1 | 23-06-2005 |
| | | | EP | 1460936 A2 | 29-09-2004 |
| | | | JP | 2005514996 T | 26-05-2005 |
| | | | US | 2003187366 A1 | 02-10-2003 |
| | | | US | 2005107718 A1 | 19-05-2005 |
| US 2005143726 | A1 | 30-06-2005 | AU | 2004312037 A1 | 21-07-2005 |
| | | | EP | 1699374 A1 | 13-09-2006 |
| | | | WO | 2005065560 A1 | 21-07-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6326177 B